# EUROPEAN PATENT APPLICATION

(11) **EP 1 870 114 A1**
(43) Date of publication of application: **26.12.2007**
(21) Application number: 06425433.7
(22) Date of filing: 23.06.2006
(51) Int. Cl.: A61L 9/20, B01D 53/88, F04D 25/08, F21V 33/00

(54) **Air purification device by means of photocatalytic reactions**

(71) Applicant: Global Engineering and Trade S.p.A., 20123 Milano (IT)
(72) Inventor: Terruzzi, Claudio, 20123 Milano (IT)
(74) Representative: Lunati, Vittoriano

(57) **Abstract**

An air purification device by means of photocatalytic reactions, comprising an aerator device (2) substantially realized by a fan comprising a stator (5) and a rotor (6), including at least one blade (6a), an active surface (3) produced on at least one of said blades (6a), which comprises photocatalytic particles, i.e., particles suitable to activate photocatalytic reactions, and at least one lighting unit (4) realized by a UV lamp integral with said rotor (5) and suitable to activate said photocatalytic particles.

## Description

The present invention relates to an air purification device by means of photocatalytic reactions, of the type specified in the preamble of claim 1.

The photocatalytic process which permits oxidation of pollutants is currently known.

Photocatalysis is based on the use of electrical charges, or electrons, provided by suitable semiconductors when they are energized by suitable electromagnetic waves.

A preferred semiconductor, for activation of the photocatalytic process, is Titanium Dioxide (TiO₂), preferably in the form of Anatase. It is energized by photons having a wavelength below 380 nm, i.e. by light having a wavelength below or within the wavelength of ultraviolet rays (UV rays).

The electrons released by the Titanium Dioxide through photon excitation provide negative electric charges which bond easily to other molecules present in the environment. In particular, they bond to the molecules of oxygen (O₂) present in the atmosphere, forming negative ions (O₂⁻ or O⁻).

The ions O₂⁻ and O⁻ are very reactive and bond to the molecules of pollutants present in the environment, in particular to nitrogen oxides (NOₓ), which are principally nitrogen monoxide and dioxide (NO or NO₂), giving rise to negative ions NO₃⁻, or to carbon monoxide (CO) producing a carbon dioxide molecule CO₂.

Not only nitrogen and carbon oxides are oxidized using the photocatalytic process, but also many other harmful substances such as: Ammonia, Benzene, Toluene, Ethyl-benzene, m-Xylene, Ozone, Sulphur Dioxide, Formaldehyde, Acetaldehyde, PMx, Methanol and Ethanol.

The photocatalytic process also causes noteworthy sterilization of the surrounding environment: bacterial activity is in fact reduced considerably.

Said process is therefore advantageously utilized to purify polluted environments; in particular it can be used to purify the air in indoor environments such as dwellings, offices, gymnasiums and the like.

In these environments, pollutants are emitted by the bodies of humans or animals, by fumes caused by the use of kitchens or the like, by emissions from cigarettes and the like, or by yet other sources.

Moreover, in heavily frequented indoor environments the sterilizing function of photocatalysis, which prevents bacteria from proliferating in these environments, is also of particular importance.

The photocatalytic process in indoor environments can be performed by different devices and instruments.

The simplest method of implementation of said process consists in coating the walls of the indoor environment to be purified with a paint or with a layer of plaster or the like containing Titanium dioxide. In this way, coated walls have the characteristic of activating photocatalytic reactions.

Nonetheless, said walls must be suitably illuminated by UV rays in order to activate said photocatalytic reactions.

As it is known, lamps intended to illuminate environments do not normally produce light with similar or lower wavelengths to ultraviolet rays. Moreover, sunlight, which comprises these light frequencies, is often filtered in indoor environments by glass and windows which, as it is known, block out the ultraviolet rays.

Consequently, the different ways of lighting indoor environments are not suitable for the purpose of activating photocatalytic reactions.

One solution to the present problem is to illuminate the environment by means of special lamps that emit UV rays. Nonetheless, said continuous lighting is neither opportune nor healthy for the human body.

It is therefore necessary to selectively illuminate the walls with UV rays. Nonetheless, said solution has considerable installation and maintenance complications, hence is not usually adopted.

Finally, photocatalysis can be implemented to purify the air of indoor environments by means of some purification devices, comprising therewithin surfaces which perform photocatalysis and produce adequate lighting.

Nonetheless, said devices are often cumbersome and unsightly, and require to be serviced by a technician from the sector.

Therefore, their application in indoor environments, especially dwellings, offices, gymnasiums and the like, is not always advantageous.

In this situation, the technical task underlying the present invention is to design an air purification system by means of photocatalytic reactions capable of substantially overcoming the aforesaid drawbacks.

Within said technical aim, an important object of the invention is to produce an air purification device by means of photocatalytic reactions which, when activated, operates constantly and is not subject to the lighting conditions of the environment.

Another important object of the invention is to create an air purification device by means of photocatalytic reactions, which is simple to install and to service and which blends visually and physically with the indoor environment.

Last but not least object of the invention is to produce an air purification device by means of photocatalytic reactions, which does not emit ultraviolet rays, or luminous rays of lower frequency, into the environment.

The technical aim and the objects specified are attained by an air purification device by means of photocatalytic reactions as claimed in the appended Claim 1.

Preferred embodiments are highlighted in the dependent claims.

Further features and advantages of the invention are better explained below in the detailed description of a preferred embodiment of the invention, with reference to the attached drawings, wherein:
**Figure 1** shows a three-dimensional view of the purification device according to the invention;
**Figure 2** highlights the section II - II of a portion of the purification device;
**Figure 3** highlights the section II - II of a first variant of the portion of the purification device shown in Figure 2; and
**Figure 4** highlights the section II - II of a second variant of the portion of the purification device shown in Figure 2; and
**Figure 5** highlights the section II - II of a third variant of the portion of the purification device shown in Figure 2.

With reference to the aforesaid Figures, the purification device according to the invention is indicated as a whole with the numeral 1.

It comprises an aerator device 2, suitable to move the air. Said aerator device 2 is substantially realized by a fan and can be of different known types: a ceiling fan for dwellings, offices, gymnasiums and the like, or yet others. The aerator device 2 can, for instance, be realized by a fan for environments such as livestock buildings.

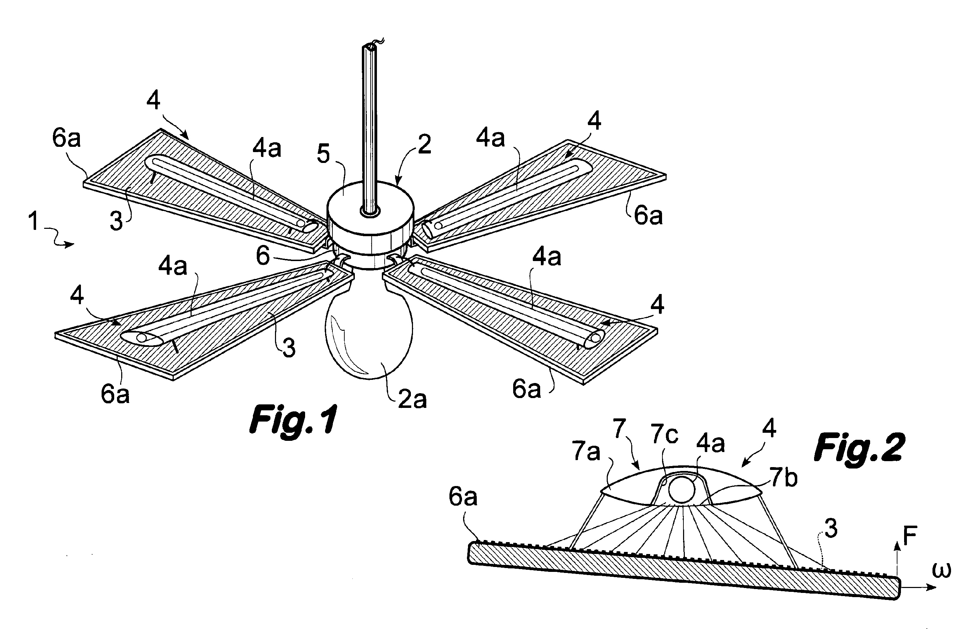

It creates, rotating in the direction of the versor **ω** (Figure 2), a continuous flow of air which is directed according to a precise direction F (Figure 2), variable according to the conformation of said fan and according to the direction of the versor w.

Said aerator device 2 therefore comprises a fixed part or stator 5 and a rotating part or rotor **6**.

The rotor 6 comprises at least one blade **6a** or the like which allows movement of the air to be purified.

Classically, ceiling fans for dwellings are provided with three or four large flat inclined blades and are supplied by electricity. Alternatively, fans with helical blades and yet others can be utilized.

Moreover, lights **2a**, realized by conventional lamps or the like, can be placed integral with the stator 5 of the ceiling fans.

The power supply of said aerator devices 2 is preferably supplied by the electricity network, which supplies power to the rotor 6 and to the lights 2a preferably by means of the same electrical connection.

The purification device 1 also comprises one or more active surfaces **3**, i.e., surfaces including photocatalytic particles suitable to activate, in the presence of suitable luminous stimuli, photocatalytic reactions.

Said photocatalytic particles are preferably composed of Titanium dioxide in the form of Anatase, or alternatively of a different semiconductor.

The active surfaces 3 are preferably produced by means of paints containing photocatalytic particles, in particular by means of siloxane paint, of known type. These paints in fact have excellent mechanical and chemical properties. Titanium Dioxide and the like are opportunely added to these paints.

The purification device 1 also comprises at least one lighting unit **4** suitable to activate photocatalysis by means of said photocatalytic particles.

The lighting unit 4 comprises lighting means **4a**, substantially realized by a lamp suitable to emit UV rays, preferably a Wood's lamp. Said lamp produces UV rays by means of a high-pressure mercury arc equipped with a barium silicate and mercury oxide filter, which allows selective emission of ultraviolet rays with a wavelength ranging from 320 to 400 nm. Moreover, said lamp preferably has a luminous intensity ranging from 10 to 0.1 W/m². Otherwise, different types of lamps 4a can be used, providing they comprise ultraviolet light waves.

The use of Titanium Dioxide in the form of Anatase combined with Wood's lamps is particularly advantageous. Said lamps in fact produce a lighting having the precise wavelength required to activate said photocatalysis. According to the known photoelectric effect, in fact, light with a higher wavelength would not be capable of activating photocatalysis, while light with a lower wavelength, with the same luminous intensity, would activate the same quantity of photocatalysis as the light utilized, however consuming a larger amount of energy.

Moreover, in the device 1 according to the invention, the active surfaces 3 are produced on the blades 6a, and the lighting units 4 are integral with the rotor 6, and preferably with the blades 6a.

Furthermore, the aerator device 1 opportunely directs the flow of air towards the lighting units 4, or in proximity thereto, so that an area of high pressure is created in proximity to said lighting units 4 and in proximity to said active surfaces 3.

The lighting units 4, and the lighting means 4a, are also preferably electrically connected to the electricity network by means of the same connection supplying power to the rotor 6 and optionally to the lights 2a.

The electrical connection between rotor 6, where the lighting units 4 are placed, and stator 5 takes place by means of known low friction or induction connections. Said known connections allow electrical contact to be implemented between portions in reciprocal movement, such as, for instance, the rotor 6 and the stator 5.

Opportunely, the lighting units 4 are placed superiorly to the blades 6a, and even more preferably a lighting unit 5 is placed above each blade 6a. In parallel with the described positioning of the lighting units 4, an active surface 3 is placed on the upper face of each blade 6a. Moreover, the blades 6a are oriented so that the flow of air created is directed upward, in the direction of the lighting units 4, i.e., in the opposite direction to known fans. Said opposite direction can be obtained by inverting the direction of rotation ω, or by inverting the orientation of the blades 6a. Said direction of rotation ω can also opportunely be variable, chosen by the user by means of known and simple devices to invert the polarity of the electrical current or the like, so that the user can choose whether to create an area of high pressure in proximity to the active surfaces 3, and obtain a more efficacious purifying action of the air, or a normal flow of air in the environment.

The position of the lighting units 4 over the blades 6a ensures that the ultraviolet rays produced by the lighting means 4a are not dispersed into the indoor environment, such as a dwelling, office or gymnasium.

For this purpose, each lighting unit 4 opportunely comprises a directional element **7** of the UV rays, substantially realized by a cover 7a not transparent to UV rays surrounding the lighting means 4a. Said directional element 7 comprises a window **7b** transparent to UV rays suitable to convey said UV rays to the active surface 3 produced on the blade 6a.

The directional element 7 preferably also has a mirror surface 7c on the inner side of said cover 7a, said surface 7c being suitable to reflect the totality of UV rays produced through the window 7b and thus onto the active surface 3.

The window 7b can be realized by material transparent to UV rays, such as quartz, specific polymers or yet other substances, or can be realized simply by an aperture. It is advantageous for the window 7b to be cut in such a way as to allow UV rays to be transmitted only to the active surface 3.

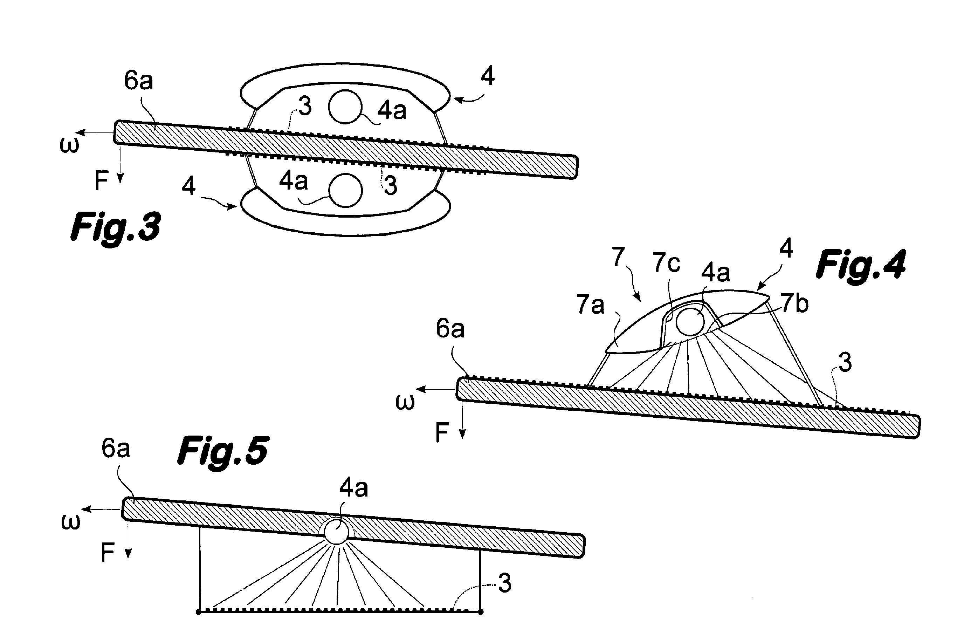

Finally, the directional element 7 preferably has an aerodynamic form suitable to ensure correct flow of the lighting unit 4 in the air, as shown in Figures 2 and 3.

If the lighting unit **4** is placed above the blades 6a and rotation of the blades 6a of the fan, or inclination thereof, is such as to direct the flow of air downward, in the opposite direction to the lighting unit, they can comprise directional elements 7, which can form a sort of baffle. I.e., these units 4 form a fan and therefore each unit 4 is in the shape of a blade or propeller, suitable to create an area of high pressure in proximity to said active surfaces 3, as shown in Figure 4. In this way, the presence of a low pressure area in proximity to the active surfaces is remedied.

Thanks to the presence of the directional elements 7 it is also possible to place the lighting units 4 under the blades 6a, without the UV rays being dispersed into the environment.

Therefore, two lighting units 4 for each blade 6a, placed superiorly and inferiorly to the blade 6a, can be provided; in this case two active surfaces for each blade 6a are opportunely produced, one on the upper surface and one on the lower surface thereof, the section of a blade according to said solution being shown in Figure 3. Said solution is indicated in the case of highly polluted environments such as livestock buildings and the like.

According to a possible variant of the device 1, the lighting units 4, shown in section in Figure 5, are inserted inside the blades 6a. In this case, the blades 6a opportunely comprise an auxiliary surface 8, suitable to receive the active surface 3. The auxiliary surface 8 is preferably positioned in the lower part of the blade 6b and is not in direct contact with the upper part of the blade 6b, in order to allow passage of the flow of air and purification thereof.

Operation of an air purification device by means of photocatalytic reactions 1, the structure of which is described above, is as follows.

The air purification device 1 is started by means of a switch or the like. Preferably, a single switch activates the entire air purification device 1.

Alternatively, different controls can be provided for the optional lights 2a, the units 4 and the aerator device 2.

The aerator device 2 allows continuous air exchange, while the lighting means 4 and the active surfaces 3 allow purification of the air by means of photocatalytic reactions.

During operation, the reciprocal position of the lighting means 4 and of the active surfaces 3 is always constant, due to the fact that both are integral with the rotor 6.

Moreover, the particular orientation of the aerator device 2, or alternatively the position of the auxiliary surface 8 or yet again the baffle conformation of the lighting unit, ensure that an area of high pressure is created in proximity to said active surfaces 3. In this area of high pressure a higher concentration of polluted particles is substantially produced; in fact, these polluted particles are presumably evenly dispersed in the air and therefore directly proportional to the quantity of air present.

The invention achieves important advantages.

The flow rates of the wall fans are in the order of hundreds of m³/hour, so that the aerator device 2 ensures that all the air in the indoor environment is continuously circulated and purified.

A fundamental advantage of the air purification device 1 is offered by the fact that it is not subject to the lighting conditions of the indoor environment, but is constantly illuminated by the lighting units 4, which have a known and precisely calibrated power in order to activate the photocatalytic activities in a consistent manner.

Moreover, the incident UV light on the blades is always constant also due to the fact that the reciprocal position of the lighting units 4 and of the active surfaces 3 does not vary, as they are not in reciprocal movement.

Another advantage is offered by the fact that the air purification device 1 does not disperse UV rays into the environment, but confines them to the active surfaces 3 present on the blades 6a; this is due both to the fact that the lighting units 4 are integral with the rotor, and to the presence of the directional elements.

Yet a further advantage is due to the fact that an area of high pressure is created in proximity to the active surfaces 3; this means that there is a higher concentration of polluted particles, which are substantially positioned evenly in the air, in proximity to said active surfaces 3, and therefore photocatalysis is more rapid and effective.

Moreover, the air purification device 1 is very easy to install, can be substantially installed in the same way as a common ceiling lamp, and blends visually and physically into the environment formed of the dwelling or the like. In fact, it is substantially visually realized by a conventional ceiling fan.

Finally, the application in which the lighting means 4 are placed superiorly to the blades 6a, and in which the direction of rotation of the aerator device 2 can be chosen, is particularly advantageous. In fact, in this case it is possible to choose whether to privilege the ventilating action of the purification device 1, directing the flow of air downward, or the purifying action of said device 1, directing the flow of air upward.

## Claims

1. Air purification device by means of photocatalytic reactions, of the type comprising: at least one active surface (3), comprising photocatalytic particles, suitable to activate photocatalytic reactions, at least one lighting unit (4) comprising lighting means (4a) suitable to activate said photocatalytic reactions on said photocatalytic particles, at least one aerator device (2) suitable to move the air in contact with said active surface (3), substantially realized by a fan comprising a stator (5) and a rotor (6), having at least one blade (6), **characterized in that** said active surface (3) is produced at the level of at least one of said blades (6a), and said lighting means (4a) are substantially realized by at least one UV lamp (4) integral with said rotor (5) and suitable to illuminate said active surfaces (3).

2. Purification device according to claim 1, wherein said lighting unit (4) is integral with at least one of said blades (6a).

3. Purification device according to claim 1, wherein said at least one lighting unit (4) comprises a directional element (7) of said UV rays substantially realized by a cover (7a) surrounding said lighting means (4) and having a window (7b) transparent to UV rays suitable to convey said UV rays selectively to said active surface (3).

4. Purification device according to claim 1, wherein said aerator device (2) is realized by a ceiling fan.

5. Purification device according to claim 4, wherein said at least one lighting unit (4) is placed superiorly to at least one of said blades (6a) and wherein said active surface (3) is produced on the upper surface of said at least one blade (6a).

6. Purification device according to claim 5, comprising one of said lighting units (4) for each of said blades (6a) and wherein each of said blades (6a) comprises said active surface (3).

7. Purification device according to claim 5, wherein said lighting units (4) substantially produce an auxiliary fan suitable to create an area of high pressure in proximity to said active surfaces (3).

8. Purification device according to claim 1, wherein said aerator device (2) is a ceiling fan with inverse rotation suitable to move the air towards said lighting unit (4).

9. Purification device according to claim 1, wherein said lighting units (4) are inserted into said blades (6a) and wherein said blades (6a) comprise an auxiliary surface (8) on which said active surface (3) is produced.

10. Purification device according to claim 1, wherein said auxiliary surface (8) is placed in the lower part of said blade (6b).

11. Purification device according to claim 1, wherein said at least one lighting unit (4) is placed both superiorly and inferiorly to at least one of said blades (6a) and wherein said active surface (3) is produced both on the upper surface and on the lower surface of said at least one blade (6a).

12. Purification device according to claim 11, comprising two of said lighting units (4) for each of said blades (6a) and wherein each of said blades comprises said active surfaces.

13. Purification device according to claim 1, wherein said lighting units (4) are realized by Wood's lamps, and wherein said photocatalytic particles are realized by particles of Titanium Dioxide in the form of Anatase.
